# EUROPÄISCHE PATENTSCHRIFT

(11) **EP 0 856 008 B1**
(45) Veröffentlichungstag und Bekanntmachung des Hinweises auf die Patenterteilung: **17.01.2001**
(21) Anmeldenummer: 96934688.1
(22) Anmeldetag: 15.10.1996
(51) Int. Cl.: C07K 14/765

(54) **VERFAHREN ZUR ABTRENNUNG VON ALBUMIN AUS SERUM DURCH IONENAUSTAUSCHCHROMATOGRAPHIE MIT MEMBRANADSORBERN**
METHOD OF SEPARATING ALBUMIN FROM SERUM BY ION-EXCHANGE CHROMATOGRAPHY WITH MEMBRANE ADSORBERS
PROCEDE POUR SEPARER DE L'ALBUMINE CONTENUE DANS DU SERUM PAR CHROMATOGRAPHIE PAR ECHANGE D'IONS AVEC DES ADSORBANTS MEMBRANAIRES

(30) Priorität: 17.10.1995 DE 19538625
(43) Veröffentlichungstag der Anmeldung: 05.08.1998
(73) Patentinhaber: SARTORIUS AG, 37075 Göttingen (DE)
(72) Erfinder: DEMMER, Wolfgang, D-37077 Göttingen (DE); GEBAUER, Klaus, Heinrich, D-52428 Jülich (DE); KULA, Maria-Regina, D-52382 Niederzier (DE); NUSSBAUMER, Dietmar, D-37079 Göttingen (DE); THOEMMES, Jörg, D-53111 Bonn (DE)
(86) Internationale Anmeldenummer: EP9604476
(87) Internationale Veröffentlichungsnummer: WO9714717

(56) Entgegenhaltungen:
- FR-A- 2 327 256
- JOURNAL OF CHROMATOGRAPHY A, Bd. 728, Nr. 1-2, 29.Mai 1996, SCIENCE NL, Seiten 129-137, XP002020303 R FREITAG ET AL.: "Controlled mixed-mode interaction chromatography on membrane absorbers"
- CHEMICAL ABSTRACTS, vol. 118, no. 16, 19.April 1993 Columbus, Ohio, US; abstract no. 155301c, K G BRIEFS & M R KULA: "Membrane chromatography" Seite 512; XP002020304 & HARNESSING BIOTECHNOLOGY. 21ST CENTURY, PROC. INT. BIOTECHNOL. SYMP. EXPO., 9TH, 1992, (LADISCH & BOSE, EDS.), ACS, WASHINGTON, DC, USA, Seiten 258-261,
- CHEMICAL ABSTRACTS, vol. 115, no. 14, 7.Oktober 1991 Columbus, Ohio, US; abstract no. 142079z, J F LACOSTE-BOURGEACQ ET AL.: "A new procedure using membrane chromatography for the valorisation of fraction IV from Kistler & Nitschmann's fractionation of blood plasma " Seite 377; XP002020305 in der Anmeldung erwähnt & CHROMATOGRAPHIA, Bd. 32, Nr. 1-2, 1991, Seiten 27-32,

## Beschreibung

Die Erfindung betrifft ein Verfahren zur Abtrennung von Albumin aus Serum durch Ionenaustauschchromatographie mit Membranadsorbern.

Das erfindungsgemäße Verfahren zeichnet sich durch eine hohe Effektivität sowie eine hohe Ausbeute und Reinheit an gewonnenem Albumin aus. Es ist auf die Isolierung von Humanserumalbumin aus humanem Serum anwendbar.

Aus menschlichem Plasma gewonnenes Albumin hat als Medikament eine breite Anwendung in der Medizin gefunden. An seine Reinheit werden deshalb hohe Anforderungen gestellt. So wird von der Pharmacopia eine Reinheit von mindestens 97 % gefordert. Neben seiner Isolierung durch kalte Ethanolfällung kommen chromatographische Aufarbeitungsverfahren zur Anwendung (J.M. Curling, "Albumin Purification by Ion Exchange Chromatography", Seiten 77 bis 91, in Methods of plasma protein fractionation, ed. J.M. Curling, Academic Press, London (1980)). In der präparativen Proteinchromatographie ist die Produktivität beziehungsweise die Trennleistung konventioneller partikulärer Adsorbermaterialien häufig durch Porendiffusionseffekte limitiert. Chromatographische Verfahren an Membranadsorbern ermöglichen hingegen durch die Struktur der mikroporösen Phase einen überwiegend konvektiven Stofftransport der Proteine zu den adsorbierenden Orten innerhalb der Membranadsorber und besitzen somit eine gesteigerte Trennleistung. J.F. Lacoste-Bourgeacq, Ch. Desneux und M. Allary ("A New Procedure Using Membrane Chromatography for the Valorization of Fraction IV from Kistler and Nitschmann's Fractionation of Blood Plasma", Chromatographia Vol. 32, No.1/2, Seiten 27 - 32 (1991)) beschreiben die chromatographische Abtrennung von Albumin aus Fraktion IV der Kistler- und Nitschmann-Fraktionierung von Blutplasma an schwach basischen (DEAE Zetaprep der Firma Cuno) und stark sauren Membranadsorbern (SP Zetaprep der Firma Cuno).
Nachteilig ist bei diesem Verfahren, daß die Albuminfraktion nach ihrer Elution vom schwach basischen Membranadsorber einer gesonderten Konditionierung unterworfen werden muß, bevor sie als Probe auf den stark sauren Membranadsorber aufgetragen werden kann. Außerdem ist nach dem Verfahren mit den verwendeten Membranadsorbern Albumin nur in relativ geringer Ausbeute und Reinheit von 66 beziehungsweise 90,2 % erhältlich.

Der Erfindung liegt daher die Aufgabe zugrunde, ein effektiveres chromatographisches Membranadsorberverfahren zur Abtrennung von Albumin aus Serum zu schaffen, daß sich durch eine hohe Produktivität sowie Ausbeute und Reinheit an abzutrennendem Albumin auszeichnet.

Die Aufgabe wird dadurch gelöst, daß die Abtrennung des Albumins an stark basischen Anionenaustauschermembranen und stark sauren Kationenaustauschermembranen durchgeführt wird. Dadurch wurde es überraschenderweise möglich, daß die aus der Anionenautauschermembran eluierte Albuminfraktion derart anfällt, daß sie direkt auf die Kationenaustauschermembran ohne gesonderte Konditionierung aufgegeben und von ihr das Albumin als Endprodukt mit hoher Ausbeute von mindestens 84 % und in hoher Reinheit von mindestens 97 % gewonnen werden kann.
Vorteilhaft ist darüber hinaus, daß bei Verwendung der stark basichen und stark sauren Ionenaustauschermembranen Verunreinigungen, an stark basischen Ionenaustauschermembranen insbesonder Immunoglobuline (IgG), vor der jeweiligen Elution der Albuminfraktion von der Anionen- und Kationenaustauschermembran mit dem gleichen Puffer entfernt werden können. Dabei können der pH-Wert und die Ionenstärke des Puffers so gewählt werden, daß unter diesen Bedingungen praktisch kein Albumin mit eluiert wird oder verloren geht.

Zur Durchführung des Verfahrens wird teildefibriniertes Frischplasma zentrifugiert um partikuläre Verunreinigungen abzutrennen. Danach wird über eine Mikrofiltrationsmembran mit einer Porengröße von 0,45µm sterilfiltriert und das Plasma durch Gelfiltration entsalzt. Nach Abtrennung der bei pH 5,4 ausgefällten Euglobuline durch Zentrifugieren wird das Plasma auf den Anionenaustauscher gegeben und durch Veränderung des pH-Wertes und der Ionenstärke eluiert. Das Eluat wird ohne weitere Manipulation auf den Kationenaustauscher gegeben. Von diesem wird das Albumin durch Veränderung des pH-Wertes und der Ionenstärke eluiert. Das Eluat enthält die gewünschte Albumin-Fraktion.

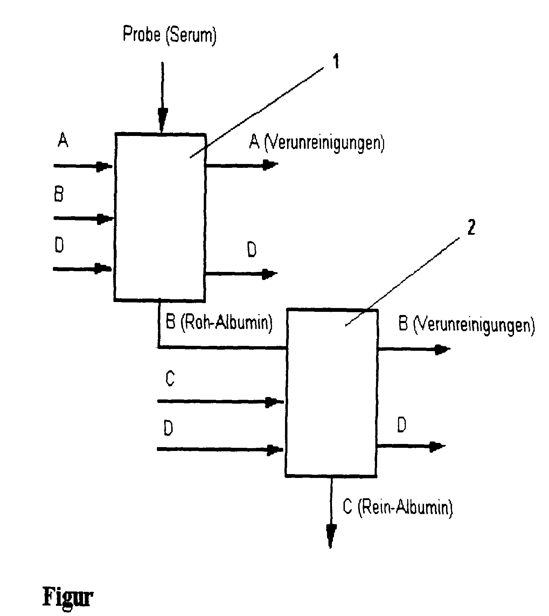

Die Erfindung wird anhand der Figur und dem nachstehenden Ausführungsbeispiel näher erläutert.
Dabei zeigt die Figur das Fließschema des erfindungsgemäßen Verfahrens.
Die stark basischen Anionenaustauschermembranen sind im Modul **1** und die stark sauren Kationenaustauschermembranen im Modul **2** enthalten. Die Pfeile auf der linken Modulseite symbolisieren den Eintritt der Puffer, die mit den Großbuchstaben **A** bis **D** gekennzeichnet sind, und die Pfeile auf der rechten und unteren Modulseite symbolisieren den Austritt der Puffer nach ihrer Konvektion durch die Membranen. In Klammern ist der Stoff angegeben, mit dem die Puffer beladen sind.

### Beispiel

Das Serum wird vorbehandelt durch:
- Auftauen einer tiefgefrorenen Serumkonserve (ca. 250 ml) bei 4°C (pH = 7,8, Leitfähigkeit (LF) = 11,3 mS/cm; Gesamtproteingehalt = 60g/l; Albumingehalt = 39 g/l),
- Zentrifugieren bei 4°C für 40 Minuten bei 15000g,
- Filtrieren über eine Mikrofiltrationsmembran mit einer Porengröße von 0,45µm (Minisart N; Sartorius AG, Göttingen),
- Entsalzen des Serums (ca. 180 ml) durch Gelfiltration über Sephadex G-25 Säule 5•50 cm (980ml) (Pharmacia Fine Chemicals, Uppsala Schweden). Flußrate =16 ml/min. Dabei erfolgt eine Umpufferung mit 25 mM Natriumacetat (NaAC)-Puffer, pH = 7,0 Nach der Gelfiltration: 290 ml Volumen; LF = 1,8 mS/cm; pH 7,5.
- Einstellen der Lösung auf pH 5,4 mit Essigsäure. Es bildet sich ein Eu-Globulin-Präzipitat.
- Zentrifugieren bei 4°C für 40 Minunten bei 15000g.
- Portionieren des Überstands bei -18°C und tiefgefrieren.

Der erhaltene Überstand hat einen Gesamtproteingehalt von 37,5 g/l. Die Albuminkonzentration beträgt 26 g/l; LF = 1,8 mS/cm.

### Durchführung der Ionenaustauschchromatographie:

- Anionenaustauscher:
   Es werden zwei stark basische Anionenaustauschermembranmodule **1**, die mit den Anionaustauschermembranen Sartobind Q 100, Charge CJ26Q (Sartorius AG, Göttingen) bestückt sind, in Reihe geschaltet und mit einer Flußrate von 24 ml/min betrieben. Das entspricht 1,22 cm/min.
   Das vorbehandelte, tiefgefroren gelagerte und bei 4°C aufgetaute Serum wurde 20 Minuten bei 4°C mit 12000g zentrifugiert, um die zu trennende Probe zu erhalten.
   Die Module wurde mit 65 ml eines Puffers **A** (Zusammensetzung: 25 mM Natriumacetat; pH = 5,4; LF = 1,8 mS/cm; Ionenstärke I = 0,025 mol/l) äquilibriert. Dann wurden 2,9 ml der Probe, entsprechend 18.9 mg Albumin/ml Adsorber auf die Anionenaustauschermembran (Adsorber) aufgepumpt.
   Anschließend wurden 28 ml Puffer **A** aufgepumpt. Das Eluat enthielt Verunreinigungen, insbesondere Immunoglobulin (IgG). Danach wurden 32 ml eines Puffers **B** (Zusammensetzung: 50 mM Natriumacetat; pH = 4,5; LF = 3,6 mS/cm; I = 0,05 mol/l) aufgepumpt. In dieser Fraktion wurde ein Rohprodukt an Humanserumalbumin (Roh-HSA) eluiert Die Albuminkonzentration war 2,15 g/l, das Volumen betrug 32 ml. Zur Regeneration des Adsorbers wurden 28 ml eines Puffers D (Zusammensetzung: 50 mM Natriumacetat; pH = 4,2 + 0,5 M NaCl; LF = 45 mS/cm; I = 0.5 mol/l) aufgepumpt. Diese Fraktion enthielt ebenfalls Verunreinigungen. Danach wurden 65 ml Puffer A aufgepumpt und ein neuer Zyklus gestartet. Ein Zyklus dauerte 6,5 Minuten.
- Kationenaustauscher:
   Es werden zwei stark saure Kationenaustauschermembranmodule **2**, die mit den Kationaustauschermembranen Sartobind S 100, Charge CJ32S (Sartorius AG, Göttingen) bestückt sind, in Reihe geschaltet und mit einer Flußrate von 24 ml/min betrieben. Das entspricht 1,22 cm/min.
   Es wurden zum Äquilibrieren 65 ml des Puffers **B** auf die Module aufgepumpt. Dann wurden 26 ml des Roh-HSA-Eluats auf die Kationenaustauschermembran (Adsorber) aufgepumpt (13,9 mg/ml Adsorber). Dann wurden 20 ml des Puffers **B** aufgepumpt. In dieser Fraktion eluierten weitere Verunreinigungen. Dann wurden 50 ml eines Puffers C (Zusammensetzung: 25 mM Natriumacetat; pH = 5,4 + 150 mM NaCl; LF = 19,8 mS/cm; I = 0,2 mol/l) aufgepumpt. In dieser Fraktion eluierte das reine HSA als Endprodukt (Albuminkonzentration = 1,1 g/l, Volumen 48,2 ml). Zur Regenerierung des Adsorbers wurden 30 ml des Puffers **D** aufgepumpt und danach ein neuer Zyklus begonnen. Ein Zyklus dauerte 8 Minuten.

Die Bestimmung des Gesamt-Proteingehaltes in den einzelnen Fraktionen wurde nach Bradford, M. (Anal. Biochem. 72, Seiten 248-254 (1976)) durchgeführt. Die Bestimmung des Albumingehaltes wurde nach Ness, A. T. et al (Clin. Chim. Acta, 12, Seiten 532-541 (1965)) durchgeführt.
Das erfindungsgemäße Verfahren ermöglicht eine Isolierung von Albumin aus Serum mit einer Reinheit von minestens 97% (Tabelle 1). Dabei wird als Reinheit das mit den Proteinbestimmungen gemessene Verhältnis von HSA zu Gesamtprotein bestimmt. Die Reinheit der Zielfraktionen sowie die mit dem erfindungsgemäßen Verfahren erzielten Ausbeuten sind in Tabelle I aufgeführt, wobei als Bezugsgröße die jeweils aufgetragene Probenmenge gewählt wurde.

**Tabelle 1:**

| Reinheit und Ausbeute der Zielfraktionen (HSA) | | |
|---|---|---|
| | Sartobind Q | Sartobind S |
| Ausbeute | 87% | 95% |
| Reinheit | 96% | 98% |

Die Produktivität des Verfahrens lag bei ca. 160g Albumin/(l Adsorber • h) für den Anionentauscher und 96g Albumin/(l Adsorber • h) für den Kationentauscher: Die in diesem Beispiel verwendeten Flußraten sind mit 1,2 cm/min vergleichsweise niedrig. Sie können ohne Verschlechterung der Trennleistung beträchtlich erhöht werden, in Abhängikeit von der Modulkonfiguration bis auf 20 cm/min, vorzugsweise bis auf 10 bis 15 cm/min.
Da sich Kapazität und Trennleistung der verwendeten stark basischen und stark sauren Ionenaustauschermembranen (Sartobind Q 100, Sartobind S 100) praktisch nicht verändern (nach mehr als 100 Cyclen wurden keine Abweichungen festgestellt), kann die Kapazitäten der Module an Anionen- und Kationenaustauschermembranen so aufeinander abgestimmt werden, daß der Prozeß durch cyclische Wiederholung der Verfahrensschritte kontinuierlich abläuft.

## Patentansprüche

1. Verfahren zur Abtrennung von Albumin aus Serum durch Membranionenaustauscherchromatographie,
dadurch gekennzeichnet, daß man
a) Serum auf die stark basische Anionenaustauschermembran aufgibt,
b) die Anionenaustauschermembran mit einem Puffer wäscht und eine erste Fraktion aus Verunreinigungen abtrennt,
c) eine Fraktion aus Roh-Albumin mit einem Puffer eluiert und das Eluat auf die stark saure Kationenaustauschermembran aufgibt,
d) die Kationenaustauschermembran mit einem Puffer wäscht und eine zweite Fraktion aus Verunreinigungen abtrennt,
e) eine Fraktion aus Rein-Albumin mit einem Puffer eluiert und
f) die Anionen- und Kationenaustauschermembran mit einem Puffer regeneriert.

2. Verfahren nach Anspruch 1,
dadurch gekennzeichnet, daß man die Schritte a) bis f) kontinuierlich durch cyclische Wiederholung durchführt.

## Claims

1. Method of separating albumin from serum by membrane ion-exchange chromatography, characterised in that
a) serum is delivered to the highly basic ion-exchanger membrane,
b) the anion-exchanger membrane is washed by a buffer and a first fraction of impurities is separated out,
c) a fraction of raw albumin is eluted by a buffer and the eluate is delivered to the highly acidic cation-exchanger membrane,
d) the cation-exchanger membrane is washed by a buffer and a second fraction of impurities is separated out,
e) a fraction of pure albumin is eluted by a buffer and
f) the anion-exchanger and cation-exchanger membranes are regenerated by a buffer.

2. Method according to claim 1, characterised in that the steps a) to f) are continuously carried out by cyclic repetition.

## Revendications

1. Procédé de séparation d'albumine à partir de sérum par chromatographie avec échange d'ions sur une membrane,
caractérisé en ce que
a) on applique du sérum sur la membrane fortement basique échangeuse d'anions,
b) on lave la membrane échangeuse d'anions avec un tampon et on sépare une première fraction d'impuretés,
c) on élue une fraction d'albumine brute avec un tampon et on applique l'éluat sur la membrane fortement acide échangeuse de cations,
d) on lave la membrane échangeuse de cations avec un tampon et on sépare une seconde fraction d'impuretés,
e) on élue une fraction d'albumine brute avec un tampon et
f) on régénère la membrane échangeuse d'anions et de cations avec un tampon.

2. Procédé selon la revendication 1, caractérisé en ce qu'on effectue les étapes a) à f) en continu par répétition cyclique.
